# EUROPEAN PATENT APPLICATION

(11) **EP 3 372 174 A2**
(43) Date of publication of application: **12.09.2018**
(21) Application number: 18275019.0
(22) Date of filing: 13.02.2018
(51) Int. Cl.: A61B 17/12, A61B 90/00, A61B 17/00

(54) **FILAMENTARY OCCLUSION ASSEMBLY**

(30) Priority: 06.03.2017 GB 201703554
(71) Applicant: Cook Medical Technologies LLC, Bloomington, IN 47404 (US)
(72) Inventor: FUREY, Aidan, 2500 Heddinge (DK); BODEWADT, Tue Thuren, 2680 Solroed Strand (DK); LARSEN, Kirsten Asser, 4440 Moerkoev (DK)
(74) Representative: Williams Powell

(57) **Abstract**

A filamentary occlusion assembly (10) includes a longitudinal filamentary element (12) of biocompatible material, such as small intestine submucosa. To provide the filamentary element (12) with both longitudinal rigidity and radiopacity, a pliable radiopaque wire (14) is wound around the filamentary element (12). It is wound such that spaced radiopaque marker regions (16) are formed by winding the radiopaque wire (14) with a small pitch. These are separated by spacer sections (18) where the radiopaque wire (14) is coiled with a much greater pitch. The filamentary occlusion assembly (10) can be delivered using a small diameter catheter (26) to fill an aneurysmal sac (22).

## Description

The present invention relates to a filamentary occlusion assembly, which can be used to fill an aneurysm or to occlude a vessel. The present invention also relates to a method of making a filamentary occlusion assembly, and to a winding apparatus for making a filamentary occlusion assembly.

There are several medical conditions that can benefit from implantation into a patient of a filler material, an embolisation coil or other device, whether temporary or permanent. Examples include the closure of blood vessels or other lumens. One condition for which such procedures can be particularly useful is in the treatment of aneurysms, where a part of a vessel wall weakens and expands outwardly to create an enlarged zone of the vessel, often having the form of a sac. This vessel expansion occurs as a result of blood pressure and tends to continue due to further and progressive weakening of the vessel wall. If left untreated, persistent pressure from the blood flow on the weakened wall tissue can lead to eventual rupture of the vessel and consequential haemorrhaging. Treatments for aneurysms have tended to focus on reducing the pressure on the weakened vessel wall, for instance by diverting blood flow or by isolating the weakened vessel wall, for instance by means of a stent graft. Another treatment method involves filling the aneurysm sac with a filler material which stops the flow of blood into the sac and as a result stops or substantially reduces the pressure on the weakened walls. The filler may be an embolisation coil, which will cause static blood around the embedded coil to clot. This blocks the sac and creates a protective barrier to prevent vessel rupture. In other methods the aneurysm may be filled with a biocompatible material, such as a hydrogel or a polysaccharide fibre, which may be biodegradable. A biodegradable filler performs the same function as an embolisation coil, that is, it fills the aneurysm sac and provides pressure protection to the weakened vessel walls, with the additional advantage of allowing remodelling of the vessel wall over time.

A useful technique involves the administration of a filamentary filler material, which can be delivered endoluminally through a small diameter catheter. The filamentary material is biocompatible and potentially also biodegradable. In many instances it is optimal to use filamentary material having a very small diameter, which enables the use of a narrow diameter delivery catheter, useful for delivery through and into small diameter vessels, for filling small aneurysm sacs, and so on. However, narrow diameter filaments can be difficult to handle, both into the delivery apparatus and from the delivery apparatus into the delivery catheter. Similar problems can also be encountered with biological or similar filamentary material, such as material made from small intestine submucosa (SIS), which can be difficult to handle especially in filamentary form. Furthermore, since such filamentary materials are generally radio-transparent, visualising these during and after deployment can be problematic.

Occlusion devices, at least portions of which are radiopaque, are disclosed in the following documents: EP 1 035 808, US 6,238,403, US 2010/0204782, US 2004/0158185, US 2003/0199887,
US 2007/0082021, and US 2004/0091543. An occlusion device is also disclosed in US 6,231,590.

The present invention seeks to provide an improved filamentary occlusion assembly, an improved method of making such, and an improved apparatus for making a filamentary occlusion assembly.

According to an aspect of the present invention, there is provided a filamentary occlusion assembly including: at least one longitudinal filamentary element of biocompatible material having a length, the filamentary element having an operational state and a first longitudinal extensibility in said operational state; at least one pliable wire of radiopaque material, the at least one wire being helically wound around the filamentary element in a plurality of at least first and second sections interposed between one another in series along the length of the filamentary element; wherein in said first sections the at least one wire is wound at a first pitch and in said second sections the at least one wire is wound at a second pitch, the first pitch being smaller than the second pitch, whereby the first sections provide spaced position markings along the filamentary element; and wherein at least the second sections of wire have a longitudinal extensibility lower than the first longitudinal extensibility of the filamentary element.

In an embodiment there are provided at least three regularly spaced position markings.

In an embodiment, the filamentary element is of a bioactive material, and may be a bioresorbable or bioabsorbable material.

In many embodiments the operational state of the filamentary element is a hydrated state.

The filamentary element may be of woven polyester, nylon or expanded polytetrafluoroethylene.

The filamentary element may be of a biological material, for example, extracellular matrix material (ECM), renal capsule membrane, dermal collagen, dura mater, pericardium, fascia lata, serosa, peritoneum and basement membrane layers. In a preferred embodiment, the filamentary element is of submucosa, for example, intestinal submucosa, stomach submucosa, urinary bladder submucosa and uterine submucosa. In a particularly preferred embodiment, the filamentary element is of small intestine submucosa.

The at least one wire may be metallic or metal, typically platinum, gold or palladium.

The at least one wire may have a diameter of about 0.05 to 0.1 mm. The filamentary element may have a diameter of about 0.12 to about 0.5 mm.

At least a first wire may be wound to provide the first sections with at least a second wire being wound to provide the second sections. Alternatively, a single wire is wound to provide both the first and second sections.

In the first sections, the wire may be coiled with no spacing between adjacent turns of coil.

The pitch between turns of the wire in the second section may be about 0.5 to 3 mm. In an embodiment, the pitch between turns of the wire in the second section is around 1 mm.

Each first section may have a length of about 0.5 mm to 3 mm. Successive first sections may be spaced from one another along the length of the filamentary element by about 1 cm to 10 cm.

According to another aspect of the present invention, there is provided a method of making a filamentary occlusion assembly, including the steps of: winding at least one pliable wire of radiopaque material around at least one longitudinal filamentary element of biocompatible material, the filamentary element having a length, an operational state and a first longitudinal extensibility in said operational state; the at least one wire being helically wound in a plurality of at least first and second sections interposed between one another in series along the length of the filamentary element; wherein in said first sections the at least one wire is wound at a first pitch and in said first sections the at least one wire is wound at a second pitch, the first pitch being smaller than the second pitch, whereby the first sections provide spaced position markings along the filamentary element; and wherein at least the second sections of wire have a longitudinal extensibility lower than the first longitudinal extensibility of the filamentary element.

The filamentary element may be dry during the winding of the wire. Preferably, the filamentary element is dried before the wire is wound thereon and the filamentary element is held in tension during drying.

In an embodiment, there are provided at least three regularly spaced position markings.

The filamentary element may be of a bioactive material, for example, it may be one of a bioresorbable or bioabsorbable material.

The filamentary element may be hydrated to be brought to an operational state.

The filamentary element may be of woven polyester, nylon or expanded polytetrafluoroethylene.

The filamentary element may be of a biological material, for example, extracellular matrix material (ECM), renal capsule membrane, dermal collagen, dura mater, pericardium, fascia lata, serosa, peritoneum and basement membrane layers. The filamentary element may be of submucosa, such as intestinal submucosa, stomach submucosa, urinary bladder submucosa and uterine submucosa. In an embodiment, the filamentary element is of small intestine submucosa.

The at least one wire may be metallic or metal. For example, the at least one wire may be of at least one of: platinum, gold and palladium.

The method may include winding at least a first wire to provide the first sections and winding at least a second wire to provide the second sections. Alternatively, the method may include winding only a single wire to provide both the first and second sections.

According to another aspect of the present invention, there is provided a winding apparatus for winding a pliable wire around at least one longitudinal filamentary element of biocompatible material having a length, the filamentary element having an operational state and a first longitudinal extensibility in said operational state; the winding apparatus including: a feed member having a lumen therein for the passage of the filamentary element therethrough, a carrier fitted to the feed member, the pliable wire being held on the carrier and dispensed therefrom onto the filamentary element, the carrier and filamentary element being rotatable relative to one another, whereby the wire is wound onto the filamentary element.

In an embodiment, the winding apparatus, includes: a drive member connectable to the filamentary element and operable to drive the filamentary element through the lumen of the feed member, wherein the drive member is operable to drive the filamentary element through the feed member, the drive member including a controller operable to vary at least one of the speed of relative rotation of the carrier and filamentary element and the speed of movement of the filamentary element past the carrier, thereby to alter the winding pitch of the wire onto the filamentary element, the controller being operable to wind the wire helically around the filamentary element in a plurality of at least first and second sections interposed between one another in series along the length of the filamentary element; wherein in said first sections the wire is wound at a first pitch and in said second sections the wire is wound at a second pitch, the first pitch being smaller than the second pitch, whereby the first sections provide spaced position markings along the filamentary element.

The carrier may be a spool.

The carrier may be rotatable around the feed member or the filamentary material may be rotatable relative to the carrier.

Other features, aspects and advantages of the apparatus disclosed herein will become apparent from the specific description which follows.

Embodiments of the present invention are described below, by way of example only, with reference to the accompanying drawings, in which:
Figure 1 shows a filamentary occlusion assembly;
Figure 2 illustrates delivery of the filamentary occlusion assembly of Figure 1 into an aneurysmal sac;
Figure 3 illustrates a method of making the filamentary occlusion assembly of Figure 1 using a winding apparatus;
Figure 4 illustrates an end view of the winding apparatus being used to make the filamentary occlusion assembly of Figure 1; and

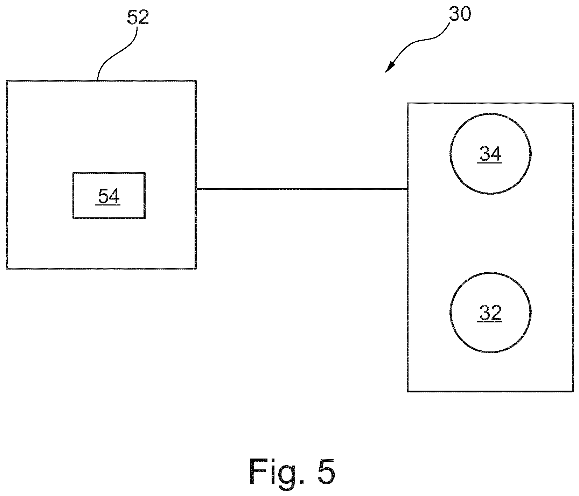

Figure 5 is a schematic illustration of a winding apparatus.

Preferred embodiments of the assembly, method and apparatus taught herein are described below and shown in the accompanying drawings. The skilled person will appreciate that the drawings are not to scale and also that minor elements and features of the various embodiments familiar in the art but not relevant to the teachings herein are not shown or described for the sake of conciseness and clarity.

As used herein, the term "bioactive" is intended to encompass the term "biodegradable", which, in turn, encompasses the terms "bioabsorbable", "bioresorbable" and "bioerodable". Any portion of a medical device of the present invention that is described herein as "bioabsorbable", "bioresorbable", or "bioerodable" will, over time, lose bulk mass by being degraded, resorbed or remodelled by normal biological processes in the body. The prefix "bio" indicates that the remodelling occurs under physiological conditions, as opposed to other remodelling processes, caused, for example, by high temperature, strong acids or bases, UV light or weather conditions. A biodegradable material has the ability naturally to disappear over time *in vivo* in accordance with any biological or physiological mechanism, such as, for example, remodelling, degradation, dissolution, chemical depolymerisation including at least acid- and base-catalysed hydrolysis and free radical-induced depolymerisation, enzymatic depolymerisation, absorption and/or resorption within the body. Typically, the material is metabolised or broken down by normal biological processes into metabolites or break-down products that are substantially non-toxic to the body and are capable of being resorbed and/or eliminated through normal excretory and metabolic processes of the body. As such, biodegradable devices do not require surgical removal.

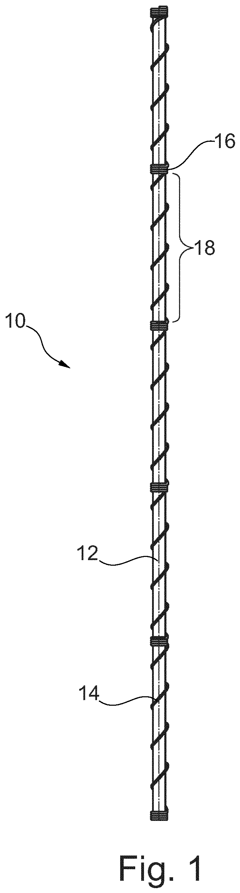

Referring first to Figure 1, a filamentary occlusion assembly 10 includes a filamentary element 12 of biocompatible material. The biocompatible material may be any biocompatible material that has an operational state, which in preferred embodiments is a hydrated state, and a first longitudinal extensibility in that operational state.

It is preferred that the filamentary element 12 is a bioactive material, which may be bioresorbable or bioabsorbable, and in a particularly preferred embodiment, the filamentary element 12 is of a biological material, such as SIS.

The filamentary element 12 in this embodiment has a length of between approximately 1 cm and 30 cm. The filamentary element in this embodiment has a diameter of about 0.15 to about 0.5 mm.

The filamentary occlusion assembly 10 includes at least one pliable wire 14 of radiopaque material (platinum in this embodiment) helically wound around the filamentary element 12. In this embodiment, a single helically wound radiopaque wire 14 is wound around the filamentary element 12 in a plurality of first and second sections interposed between one another in series along the length of the filamentary element 12. In the first sections, the radiopaque wire 14 is wound at a first, smaller pitch to form a plurality of marker regions 16. The marker regions 16 are spaced from one another along the length of the filamentary element 12 by the second so-called spacer sections 18 in which the radiopaque wire 14 is wound at a greater pitch than that of the first sections 16. At least the second sections 18 of wire have a longitudinal extensibility lower than the first longitudinal extensibility of the filamentary element 12.

Generally the radiopaque wire 14 is coiled with no or minimal spacing between adjacent turns of coil in the first sections 16. Suitable biocompatible materials for the filamentary element 12 are generally radiotranslucent. The radiopaque wire 14 in the marker regions 16 provides a means of visualising the filamentary element 12 during delivery to facilitate the deployment procedure.

Each first section 16 is preferably around 0.5 to 3 mm in length. This provides sufficient density of radiopaque wire 14 to form radiopaque marker sections 14 spaced along the filamentary element 12. The second spacer regions 18 may be between approximately 1 and 10 cm in length. The pitch of the turns of the radiopaque wire 14 in the second spacer sections 18 may typically be from 0.5 to 3 mm, although a pitch of around 1 mm may be appropriate. "Pitch" is used to mean the length of one complete helix turn, measured parallel to the axis of the helix. By minimising the amount of radiopaque wire 14 found within the second spacer sections 18, the marker regions 14 should be clearly separated from one another under X-ray visualisation such that they may be used as distance markers to aid delivery of the device *in vivo.*

The radiopaque wire 14 should be as thin as possible so as not to increase the overall diameter of the filamentary occlusion assembly 10 too much. Typically the radiopaque wire will have a diameter within the range of about 0.002 to 0.004 inches (0.05 to 0.1 mm). The filamentary occlusion assembly 10 preferably has an overall diameter no greater than 0.018 inches (0.5 mm).

Of course, in modifications of the above described embodiment, the filamentary element 12 could be of any suitable material. This could be a biological material, such as extracellular matrix material (ECM), renal capsule membrane, dermal collagen, dura mater, pericardium, fascia lata, serosa, peritoneum and basement membrane layers. It could be of submucosa, such as intestinal submucosa, stomach submucosa, urinary bladder submucosa and uterine submucosa. In other modifications, the filamentary element 12 could be of woven polyester, nylon or expanded polytetrafluoroethylene.

Any suitable radiopaque material could be used for the radiopaque wire 14. Typically it might be a metal, such as palladium or gold. The skilled person will be aware of other suitable materials.

In the embodiment illustrated in Figure 1, a single wire is used to provide both the first sections (the marker regions 16) and the second spacer sections 18. In a modification, at least a first radiopaque wire 14 is wound to provide the first sections 16, and at least a second radiopaque wire 14 is wound to provide the second sections 18.

Figure 1 illustrates an embodiment where the pitch of the radiopaque wire 14 is constant within the second spacer sections 18. It will be clear to the skilled person that the pitch of the coil between the marker regions 16 need not be constant along the length of the filamentary occlusion assembly 10.

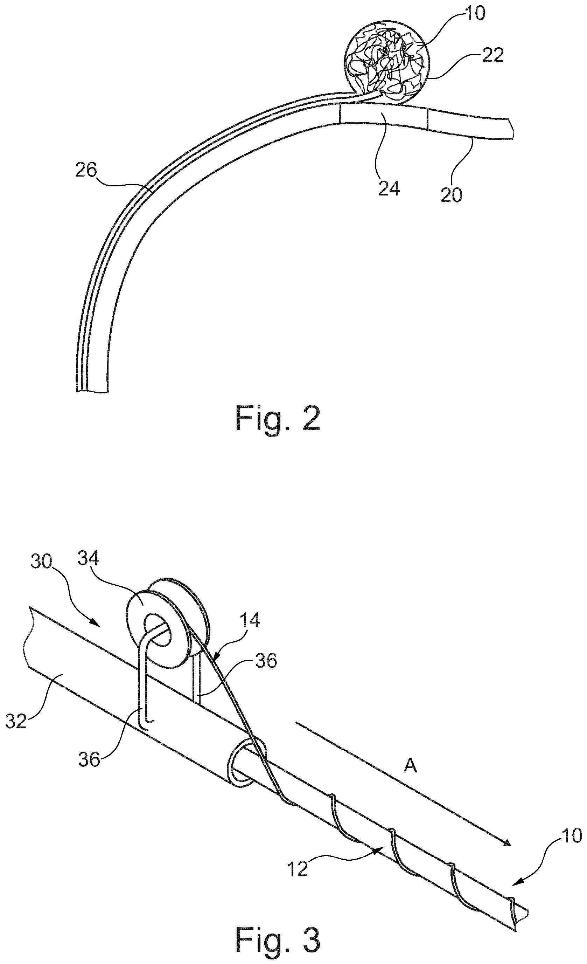

The above-described filamentary occlusion assembly 10 is envisaged primarily for use in neurological applications, for example for treatment of neuro-aneurysm, embolisation of arterio-venous malformations, or for vessel embolisation. Figure 2 illustrates a vessel 20 having an aneurysm 22 therein. The aneurysm 22 forms a sac to one side of the vessel 20. A support structure, typically a stent, 24 is shown positioned across the neck of the aneurysm and is used to hold the filamentary occlusion assembly 10 within the aneurysm sac 22.

A catheter 26 is positioned within the vessel 20, such that its distal end is disposed within the aneurysm 22. The filamentary occlusion assembly 10 is delivered through the catheter 26 into the aneurysm sac to fill the aneurysm 22 using flow and drag and is thereby pulled through the catheter 26 in a known manner. Once sufficient material has been delivered, the catheter 26 can be removed from the patient. The stent 24 may in some instances be removed, but in other cases is left within the patient. This could be permanent but could also be made of a biodegradable or bioresorbable material.

The filamentary occlusion assembly 10 is intended to fill at least a significant part of the volume of the aneurysm sac 22 so as to stop the flow of blood into the aneurysm 22 and as a result reduce the pressure of blood on the weakened vessel walls of the aneurysm. In the case of a bioresorbable or bioabsorbable filamentary element 12, this will eventually be resorbed or absorbed, typically after a sufficient period to allow recovery of the weakened vessel wall and remodelling of the vessel. In other cases the fibrous material remains permanently within the aneurysm sac, effectively closing this off. The radiopaque wire 14 would simply become embedded within the vessel walls during remodelling without causing any harm to the patient.

The aneurysm 22 need not be completely filled with the filamentary occlusion device 10. Some material for the filamentary element 12, such as SIS will expand in blood, thereby filling the aneurysm 22 over time. In other cases, a relatively loose arrangement for the filamentary occlusion assembly 10 within the aneurysm sac 22 will be sufficient to divert blood flow away from the aneurysm sac 22 and also to promote thrombosis within the aneurysm 22, which will cause natural closure thereof and effective repair of the vessel 20.

For delivery into a patient, the filamentary occlusion assembly 10 is hydrated. Ordinarily this would cause the filamentary element 12 to lose its longitudinal integrity and become flexible and elastic. However, the presently described device includes a radiopaque wire 14, which is substantially inextensible. Not only does this provide spaced radiopaque marker regions 16 as described above, but also longitudinal rigidity to the filamentary occlusion assembly 10.

In order to make the above-described filamentary occlusion assembly 10, at least one pliable wire of radiopaque material 14 (for example, platinum) is wound around the longitudinal filamentary element 12 of biocompatible material, such as small intestine submucosa. Suitable materials for the filamentary element 12 include those mentioned above, and tend to be relatively rigid when dry, but relatively flexible and elastic when wet.

The filamentary element 12 is preferably dry during winding of the wire 14. Then, when the filamentary occlusion assembly 10 is hydrated for delivery, the filamentary element 12 can swell and the radiopaque wire 14 becomes stably embedded into the outer surface of the filamentary element 12. In some embodiments, the filamentary element 12 is held in tension during drying. Drying the filamentary element 12 whilst it is held in tension prevents it elongating further when it is hydrated prior to deployment. This ensures that the radiopaque wire 14 remains properly coiled around the filamentary element 12 during deployment, and therefore preserves the spacing of the marker regions 16.

Figure 3 illustrates a method of making the filamentary occlusion assembly 10 using a winding apparatus 30, which dispenses the pliable radiopaque wire 14 around the filamentary element 12.

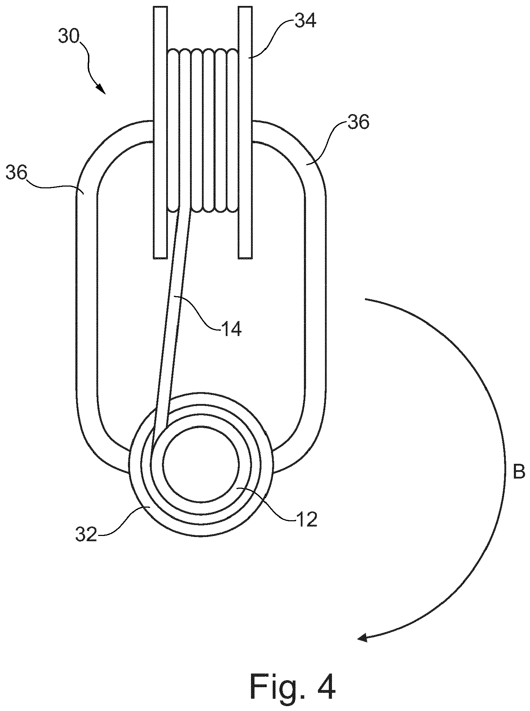

As shown in Figures 3 and 4, the winding apparatus 30 includes a feed member having a lumen therein (in this instance a cannula 32) for the passage of the filamentary element therethrough (illustrated by Arrow A in Figure 3). A carrier, which is preferably a spool 34, is fitted to the feed member 32, the pliable wire 14 being held on the carrier 34 and dispensed therefrom onto the filamentary element 12. The carrier 34 and the filamentary element 10 are rotatable relative to one another (illustrated by Arrow B in Figure 4), whereby the wire 14 is wound onto the filamentary element 10. The carrier 34 may be rotatable around the feed member 32 or the filamentary material 10 may be rotatable relative to the carrier 34.

In the embodiment of winding apparatus illustrated in Figures 3 and 4, the spool 34 is rotatably mounted by a pair of mounting arms 36 to the cannula 32 so that the spool 34 is in a fixed relationship with the cannula 32. Radiopaque wire 14 is dispensed from the spool by rotation thereof. The filamentary element 12 can move through the lumen of the cannula 32 in a longitudinal direction (A), either by being pulled through the lumen, or by the cannula 32 being pulled along the filamentary element 12 in a direction opposite the dispensing direction of the radiopaque wire 14 from the spool 34.

Furthermore, the relative rotation (B) between the cannula 32 and the filamentary element 12 whilst the filamentary element is being pulled through the lumen of the cannula 32 causes the radiopaque wire 14 to wrap around the filamentary element 12 in coils. The distance between adjacent coils can be varied by altering the speed of the relative rotation of the cannula 32 (and thus the spool 34) and the filamentary element 12, and/or altering the speed of the relative longitudinal movement of the cannula 32 (and thus the spool 34) and the filamentary element 12.

The winding apparatus 30 may include a drive member connectable to the filamentary element 12 and operable to drive the filamentary element 12 through the lumen of the feed member 32. The drive member is operable to drive the filamentary element 12 through the feed member 32 and includes a controller operable to vary at least one of the speed of relative rotation of the carrier 34 and filamentary element 12 and the speed of movement of the filamentary element 12 past the carrier 34. This results in alteration of the winding pitch of the wire 14 onto the filamentary element 12. The controller is operable to wind the wire 14 helically around the filamentary element 12 in a plurality of at least first sections 16 and second sections 18 interposed between one another in series along the length of the filamentary element 12.

It can be seen from the above description that a filamentary occlusion assembly 10 is provided, which can be deployed more easily than prior art devices. The radiopaque wire 14 provides not only a means of visualising the filamentary element 12 during deployment, but also longitudinal integrity to the filamentary element 12. The marker regions 16 are regularly spaced so that the progress of deployment can be easily tracked. The disclosed winding apparatus 30 provides a simple way of winding a pliable wire 14 onto the filamentary element 12 at a given pitch, and for varying the pitch where required.

All optional and preferred features and modifications of the described embodiments and dependent claims are usable in all aspects of the invention taught herein. Furthermore, the individual features of the dependent claims, as well as all optional and preferred features and modifications of the described embodiments are combinable and interchangeable with one another.

The disclosures in United Kingdom patent application 1703554.4, from which this application claims priority, and in the abstract accompanying this application are incorporated herein by reference.

## Claims

1. A filamentary occlusion assembly including:
at least one longitudinal filamentary element of biocompatible material having a length, the filamentary element having an operational state and a first longitudinal extensibility in said operational state;
at least one pliable wire of radiopaque material, the at least one wire being helically wound around the filamentary element in a plurality of at least first and second sections interposed between one another in series along the length of the filamentary element, wherein there are at least three first sections;
wherein in said first sections the at least one wire is wound at a first pitch and in said second sections the at least one wire is wound at a second pitch, the first pitch being smaller than the second pitch, whereby the first sections provide regularly spaced position markings along the filamentary element; and
wherein at least the second sections of wire have a longitudinal extensibility lower than the first longitudinal extensibility of the filamentary element.

2. A filamentary occlusion assembly as claimed in claim 1, wherein the at least one wire has a diameter of about 0.05 to 0.1 mm.

3. A filamentary occlusion assembly as claimed in claim 1 or 2, wherein the filamentary element has a diameter of about 0.12 to about 0.5 mm.

4. A filamentary occlusion assembly as claimed in claim 1, 2 or 3, wherein in the first sections the wire is coiled with no spacing between adjacent turns of coil; and/or wherein the pitch between turns of the wire in the second section is 0.5 to 3 mm, optionally wherein the pitch between turns of the wire in the second section is around 1 mm.

5. A filamentary occlusion assembly as claimed in any preceding claim, wherein each first section has a length of about 0.5 mm to 3 mm.

6. A filamentary occlusion assembly according to any preceding claim, wherein successive first sections are spaced from one another along the length of the filamentary element by about 1 cm to 10 cm.

7. A method of making a filamentary occlusion assembly, including the steps of:
winding at least one pliable wire of radiopaque material around at least one longitudinal filamentary element of biocompatible material, the filamentary element having a length, an operational state and a first longitudinal extensibility in said operational state;
the at least one wire being helically wound in a plurality of at least first and second sections interposed between one another in series along the length of the filamentary element;
wherein in said first sections the at least one wire is wound at a first pitch and in said first sections the at least one wire is wound at a second pitch, the first pitch being smaller than the second pitch, whereby the first sections provide spaced position markings along the filamentary element; and
wherein at least the second sections of wire have a longitudinal extensibility lower than the first longitudinal extensibility of the filamentary element.

8. A method according to claim 7, wherein the filamentary element is dry during the winding of the wire, optionally wherein the filamentary element is dried before the wire is wound thereon, the filamentary element being held in tension during drying.

9. A filamentary occlusion assembly or a method as claimed in any preceding claim, wherein the filamentary element is of a bioactive material, a bioresorbable material or a bioabsorbable material.

10. A filamentary occlusion assembly or a method as claimed in any preceding claim, wherein the operational state of the filamentary element is a wetted state.

11. A filamentary occlusion assembly or a method as claimed in any preceding claim, wherein the filamentary element is of woven polyester, nylon or expanded polytetrafluoroethylene, wherein the filamentary element is of a biological material, wherein the filamentary element is of at least one of: extracellular matrix material (ECM), renal capsule membrane, dermal collagen, dura mater, pericardium, fascia lata, serosa, peritoneum and basement membrane layers, wherein the filamentary element is of submucosa, wherein the filamentary element is of at least one of: intestinal submucosa, stomach submucosa, urinary bladder submucosa and uterine submucosa, and/or wherein the filamentary element is of small intestine submucosa.

12. A filamentary occlusion assembly according to any preceding claim, wherein the at least one wire is metallic or metal, optionally wherein the at least one wire is of at least one of: platinum, gold and palladium.

13. A filamentary occlusion assembly or a method as claimed in any preceding claim, wherein at least a first wire is wound to provide the first sections and at least a second wire is wound to provide the second sections.

14. A filamentary occlusion assembly or a method as claimed in any of claims 1 to 12, wherein a single wire is wound to provide both the first and second sections.

15. Winding apparatus for winding a pliable wire around at least one longitudinal filamentary element of biocompatible material having a length, the filamentary element having an operational state and a first longitudinal extensibility in said operational state; the winding apparatus including:
a feed member having a lumen therein for the passage of the filamentary element therethrough,
a carrier fitted to the feed member, the pliable wire being held on the carrier and dispensed therefrom onto the filamentary element,
the carrier and filamentary element being rotatable relative to one another, whereby the wire is wound onto the filamentary element.
